# EUROPEAN PATENT APPLICATION

(11) **EP 3 586 749 A1**
(43) Date of publication of application: **01.01.2020**
(21) Application number: 19181560.4
(22) Date of filing: 20.06.2019
(51) Int. Cl.: A61B 6/00, A61B 6/02, A61B 6/10, A61B 10/02

(54) **SYSTEM AND METHOD FOR CONTACT MANAGEMENT OF A BIOPSY APPARATUS**

(30) Priority: 21.06.2018 US 201816014374
(71) Applicant: General Electric Company, Schenectady, NY 12345 (US)
(72) Inventor: VANCAMBERG, Laurence, 78530 BUC (FR)
(74) Representative: Fennell, Gareth Charles

(57) **Abstract**

A system 10 for management of a biopsy apparatus 12 is provided. The system 10 includes an imaging device 14 and a controller 16. The imaging device 14 is operative to obtain one or more images 18, 20, 22, 24, 26, 28, 30 of an object 32 to be biopsied by the biopsy apparatus 12. The controller 16 is in electronic communication with the imaging device 14 and operative to receive the one or more images 18, 20, 22, 24, 26, 28, 30; and to generate a contour 36 of the object 32 based at least in part on the one or more images 18, 20, 22, 24, 26, 28, 30. The controller 16 is further operative to determine, based at least in part on the contour 36, if the biopsy apparatus 12 will contact the object 32 during biopsy of the object 32; and to generate an indicator 38, 40 upon determining that the biopsy apparatus 12 will contact the object 32.

## Description

### BACKGROUND

### TECHNICAL FIELD

Embodiments of the invention relate generally to medical biopsy procedures, and more specifically, to a system and method for management of a biopsy apparatus during a biopsy procedure.

### DISCUSSION OF ART

Many medical biopsy procedures concern obtaining a biopsy sample, i.e., a tissue sample, from a body part of a patient suspected as being cancerous, and then testing the biopsy sample for indications that the body part contains cancer cells. In many breast biopsy procedures, a needle is inserted into the breast via a biopsy apparatus. In such procedures, the biopsy apparatus typically guides the needle to a suspect region, i.e., a target biopsy site, within the breast via an x-ray imaging system, e.g., a digital tomosynthesis imaging system. The patient's breast is usually positioned on a breast support located between the detector and the ray source, and then held/compressed in place against the breast support by a compression plate.

Many breast biopsy procedures use needles of predetermined lengths, e.g., short, medium, and/or long, with the size of the compressed breast and location of the biopsy site determining the length of the needle used. In other words, the distance between the biopsy site and the border of the breast, i.e., the surface, when in a compressed state, determines which size needle is used. Many traditional imaging systems, however, have fields of view ("FOV") which are too small to accurately depict the border of the breast in combination with the biopsy site. Additionally, the biopsy site is often not visible to a radiologist performing the biopsy. Thus, it is often difficult for a radiologist to accurately calculate the distance from the breast border to the biopsy site. Accordingly, many radiologists often use the longest needle available to improve the odds of reaching the biopsy site. Use of long needles, however, may increase the risk of the needle deflecting and/or decrease the accuracy of correctly guiding the needle to the biopsy site.

Further, in certain circumstances, e.g., a breast larger than the compression plate and/or an unusual positioning of the breast between the breast support and the compression plate, some of the breast tissue may remain uncompressed. In such circumstances, the distance between the biopsy site and the breast border may be different than expected, which, in turn, may result in the needle penetrating the breast sooner than expected by both the physician and/or the patient, and/or with part of the biopsy apparatus contacting the breast in an unexpected manner such that the needle may miss the biopsy site.

What is needed, therefore, is an improved system and method for management of a biopsy apparatus during a biopsy procedure.

### BRIEF DESCRIPTION

In an embodiment, a system for management of a biopsy apparatus is provided. The system includes an imaging device and a controller. The imaging device is operative to obtain one or more images of an object to be biopsied by the biopsy apparatus. The controller is in electronic communication with the imaging device and operative to receive the one or more images; and to generate a contour of the object based at least in part on the one or more images. The controller is further operative to determine, based at least in part on the contour, if the biopsy apparatus will contact the object during biopsy of the object; and to generate an indicator upon determining that the biopsy apparatus will contact the object.

In another embodiment, a method for management of a biopsy apparatus is provided. The method includes obtaining one or more images of an object to be biopsied by the biopsy apparatus; and receiving the one or more images at a controller. The method further includes generating, via the controller, a contour of the object based at least in part on the one or more images; determining, via the controller, if the biopsy apparatus will contact the object during biopsy of the object based at least in part on the contour; and generating, via the controller, a contact indicator upon determining that the biopsy apparatus will contact the object.

In yet another embodiment, a non-transitory computer readable medium storing instructions is provided. The stored instructions adapt a controller to receive one or more images of an object to be biopsied by a biopsy apparatus; and to generate a contour of the object based at least in part on the one or more images. The stored instructions further adapt the controller to determine, based at least in part on the contour, if the biopsy apparatus will contact the object during biopsy of the object; and to generate a contact indicator upon determining that the biopsy apparatus will contact the object.

### DRAWINGS

The present invention will be better understood from reading the following description of non-limiting embodiments, with reference to the attached drawings, wherein below:
FIG. 1 is a perspective view of a system for management of a biopsy apparatus, in accordance with an embodiment of the present invention;
FIG. 2 is a diagram depicting the generation of a model of an object to be biopsied by the system of FIG 1, wherein the model is generated from a stereo pair of images of the object, in accordance with an embodiment of the present invention;
FIG. 3 is a diagram depicting the generation of a model of an object to be biopsied by the system of FIG 1, wherein the model is generated from one or more images acquired via tomosynthesis, in accordance with an embodiment of the present invention;
FIG. 4 is a diagram depicting the generation of a model of an object to be biopsied by the system of FIG 1, wherein the model is generated from a scout image of the object, in accordance with an embodiment of the present invention;
FIG. 5 is a diagram of a contour superimposed onto the object, wherein the contour is generated from the model of FIGS. 2, 3, and/or 4, in accordance with an embodiment of the present invention;
FIG. 6 is a diagram of a simulation of a biopsy attempt of the object that utilizes the contour of FIG. 5 to determine if contact will occur between the biopsy apparatus and the object, in accordance with an embodiment of the present invention; and
FIG. 7 is a diagram of a trajectory for the biopsy apparatus of FIG. 1, wherein the trajectory is generated by a controller of the system of FIG. 1, in accordance with an embodiment of the present invention.

### DETAILED DESCRIPTION

Reference will be made below in detail to exemplary embodiments of the invention, examples of which are illustrated in the accompanying drawings. Wherever possible, the same reference characters used throughout the drawings refer to the same or like parts, without duplicative description.

As used herein, the terms "substantially," "generally," and "about" indicate conditions within reasonably achievable manufacturing and assembly tolerances, relative to ideal desired conditions suitable for achieving the functional purpose of a component or assembly. As used herein, "electrically coupled", "electrically connected", and "electrical communication" mean that the referenced elements are directly or indirectly connected such that an electrical current may flow from one to the other. The connection may include a direct conductive connection, i.e., without an intervening capacitive, inductive or active element, an inductive connection, a capacitive connection, and/or any other suitable electrical connection. Intervening components may be present. Additionally, the terms "collide" and/or "collision", as used herein with respect to a biopsy apparatus and an object to be biopsied, refer to a situation/scenario/event where a part of the biopsy apparatus is compressing the object. The terms "compress" and/or "compressing", as used herein with respect to a biopsy apparatus and an object to be biopsied, mean to distort the matter/tissue of the object in an unexpected and/or undesirable manner. The terms "contact" and "contacting", as used herein with respect to a biopsy apparatus and an object to be biopsied, refers to a situation/scenario/event where the biopsy apparatus touches the matter/tissue of the object in an unexpected and/or undesirable manner.

Further, while the embodiments disclosed herein are described with respect to a breast biopsy system and procedure, it is to be understood that embodiments of the present invention may be applicable to other types of biopsy procedures. Further still, as will be appreciated, embodiments of the present invention related imaging systems may be used to analyze tissue generally and are not limited to human tissue.

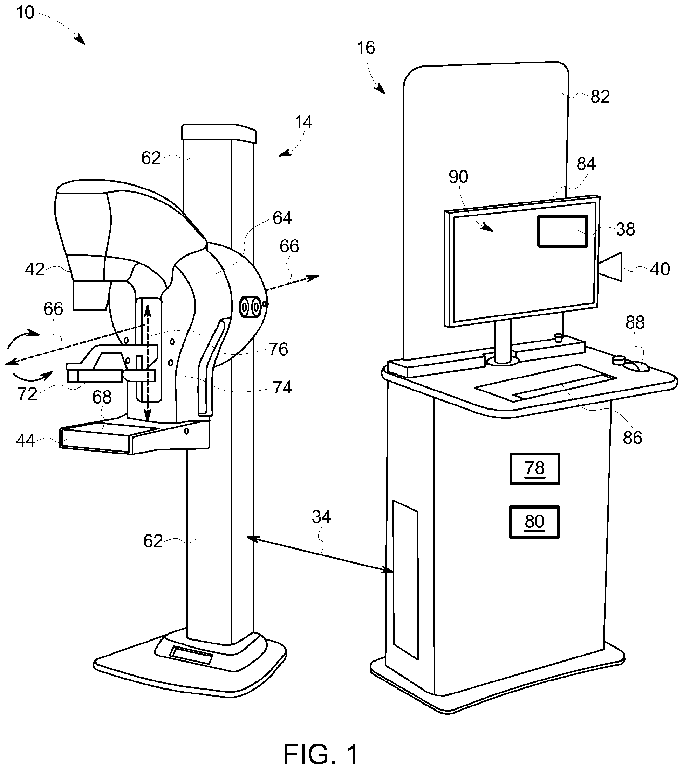

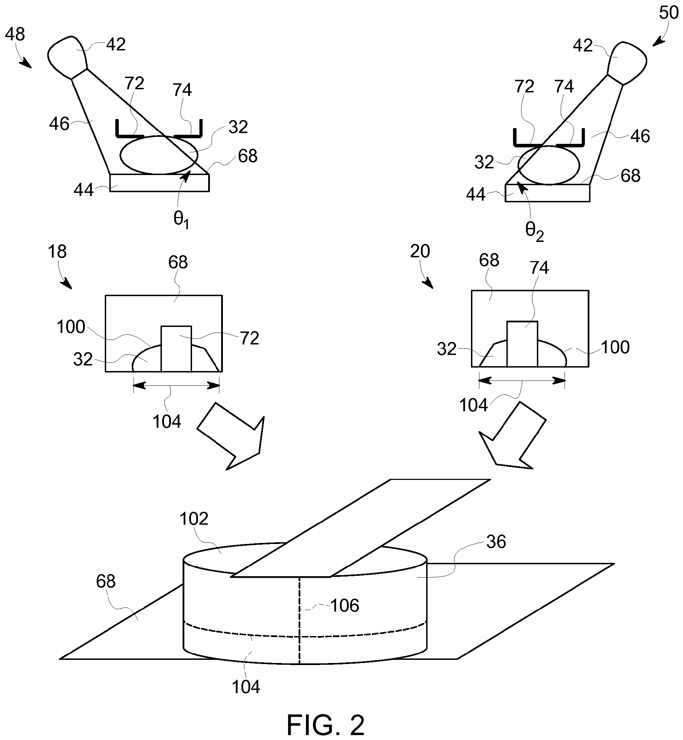

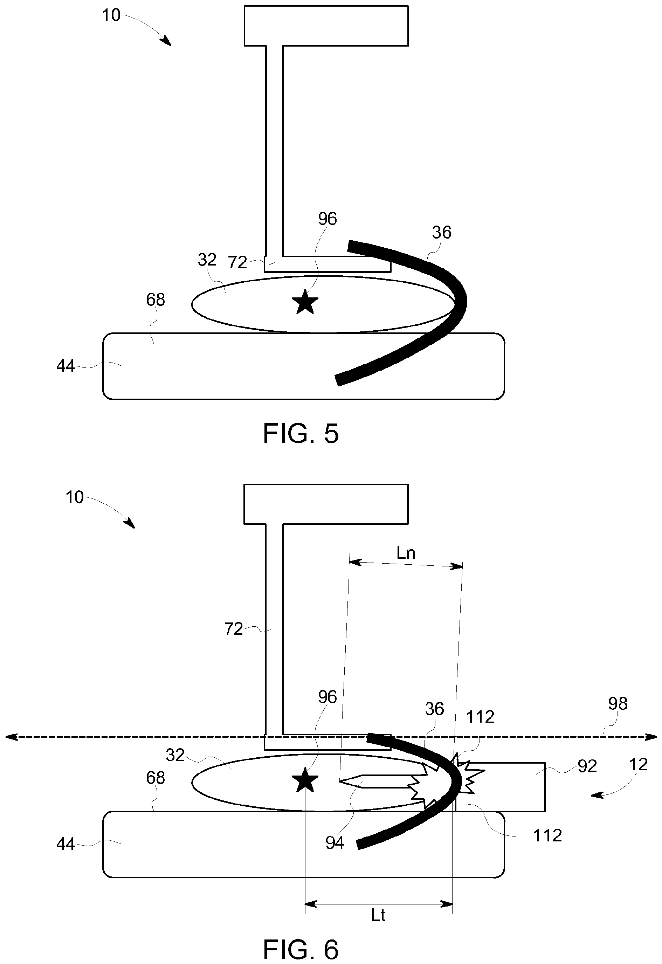

Referring now to FIG. 1, the major components of a system 10 for management of a biopsy apparatus/tool/robot 12 (FIG. 6) in accordance with an embodiment of the present invention are shown. As will be appreciated, in embodiments, the system 10 includes an imaging device/system 14 and a controller 16. The imaging device 14 is operative to obtain one or more images 18, 20, 22, 24, 26, 28, 30 (FIGS. 2, 3, and 4) of an object 32, e.g., a human breast or other body part, to be biopsied by the biopsy apparatus 12. The controller 16 electronically communicates with the imaging device 14, via data link 34, which, in embodiments, may be a wired and/or wireless connection. As will be explained in greater detail below, the controller 16 is operative to receive the one or more images 18, 20, 22, 24, 26, 28, 30, and to generate a contour/frontier/mask/virtual border 36 of the object 32, based at least in part on the one or more images 18, 20, 22, 24, 26, 28, 30. The controller 16 is further operative to determine, based at least in part on the contour 36, if the biopsy apparatus 12 will compress/contact the object 32 during biopsy of the object 32, and, upon determining that the biopsy apparatus 12 will compress/contact the object 32, generate a contact indicator 38 and/or 40.

As shown in FIG. 1, the imaging device 14 includes a radiation source/emitter 42 and a radiation detector 44. The radiation source 42 is operative to emit radiation rays 46 (FIGS. 2, 3, and 4) and is selectively adjustable between one or more positions 48, 50, 52, 54, 56, 58, and 60 (FIGS. 2, 3, and 4), e.g., the radiation source 42 may be mounted to a stand/support 62 via a rotatable mount 64 such that the radiation source 42 rotates about a longitudinal axis 66. The radiation detector 44 is operative to receive the radiation rays 46 and has a surface 68 that defines an imaging region (depicted as the top-down view of the surface 68 in images 18, 20, 22, 24, 26, 28, and 30 of FIGS. 2, 3, and 4). In embodiments, the imaging device 14 may include one or more paddles 72 and 74, e.g., a compression plate, mounted to the stand 62 and slidably adjustable along axis 76 (and/or other axis/direction) so as to compress and/or restrain the object 32 against the surface 68. In embodiments, the imaging device 14 may form part of/be a mammography imaging system/device.

In embodiments, the controller 16 may be a workstation having at least one processor 78 and a memory device 80. In other embodiments, the controller 16 may be embedded / integrated into one or more of the various components of the imaging system 10 disclosed above. In embodiments, the controller 16 may be in electrical communication with the radiation source 42, radiation detector 44, the paddles 72 and 74, and/or the biopsy apparatus 12 via link 34. As will be appreciated, in embodiments, the connection 34 may be a wireless connection. In embodiments, the controller 16 may include a radiation shield 82 that protects an operator of the system 10 from the radiation rays 46 emitted by the radiation source 42. The controller 16 may further include a display 84, a keyboard 86, mouse 88, and/or other appropriate user input devices, that facilitate control of the system 10 via a user interface 90.

Referring briefly to FIG. 6, in embodiments, the biopsy apparatus 12 may include a body 92 operative to direct/guide/support a probe 94, e.g., a biopsy tool/needle. In embodiments, the probe 94 may be a hookwire for surgical site pre-localization. The body 92 is operative to guide the probe 94, having a length Ln, to a target site 96 within the object 32. In embodiments, the biopsy apparatus 12 may be manually positioned/guided by an operator of the system 10 such that the probe 94 reaches the target site 96. In other embodiments, the biopsy apparatus 12 may be automatically positioned/guided by the controller 16 such that the probe 94 reaches the target site 96, e.g., the biopsy apparatus 12 may be an autonomous robot. As shown in FIG. 6, the biopsy apparatus 12 may be positioned such that the probe 94 penetrates the object 32 from the side with respect to the orientation of the paddles 72 and 74, e.g., in a direction parallel to a horizontal axis 98 of the paddles 72 and 74. As will be understood, however, in other embodiments the biopsy apparatus 12 may be positioned such that the probe 94 penetrates the object 32 at an angle with respect to axis 98.

Referring now to FIG. 2, in embodiments, the one or more images used by the controller 16 to generate the contour 36 (FIGS. 5 and 6) may be a pair, e.g., a stereo pair such as images 18 and 20. In such embodiments, the object 32 may be positioned onto the surface 68 of the detector 44 and compressed by the paddles 72 and 74. A first image 18 is acquired with the radiation source 42 at a first position 48 with the rays 46 intercepting the detector 44 at a first angle θ₁. A second image 20 is then acquired with the radiation source 42 at a second position 50 with the rays 46 intercepting the detector 44 at a second angle θ₂ which is different than θ₁. For example, in embodiments, θ₁ and θ₂ may have the same magnitude but different signs. As shown in FIG. 2, the images 18 and 20 may capture/include the border 100 of the object 32. As will be appreciated, the controller 16 may generate the contour 36 by generating an estimate/model 102 of the object 32 based on detection of the border 100. As will be appreciated, embodiments of the present invention may utilize a variety of image processing techniques to detect the border 100. For example, in embodiments, the controller 16 may be able to determine a radius and/or diameter 104 of the object 32 from the images 18 and 20, and then model the object 32 as a cylinder, or other appropriate shape, e.g., a sphere, having the determined radius and/or diameter 104 and a height 106 based on known or estimated values of the object 32. As will be understood, the contour 36 may be based at least in part on the border/external surface of the model 102, e.g., cylinder. While images 18 and 20 are depicted herein as a stereo pair, it will be understood that, in embodiments, the images 18 and 20 need not be a stereo pair, i.e., images 18 and 20 may be two images taken at angles of differing magnitudes not having a stereo relationship.

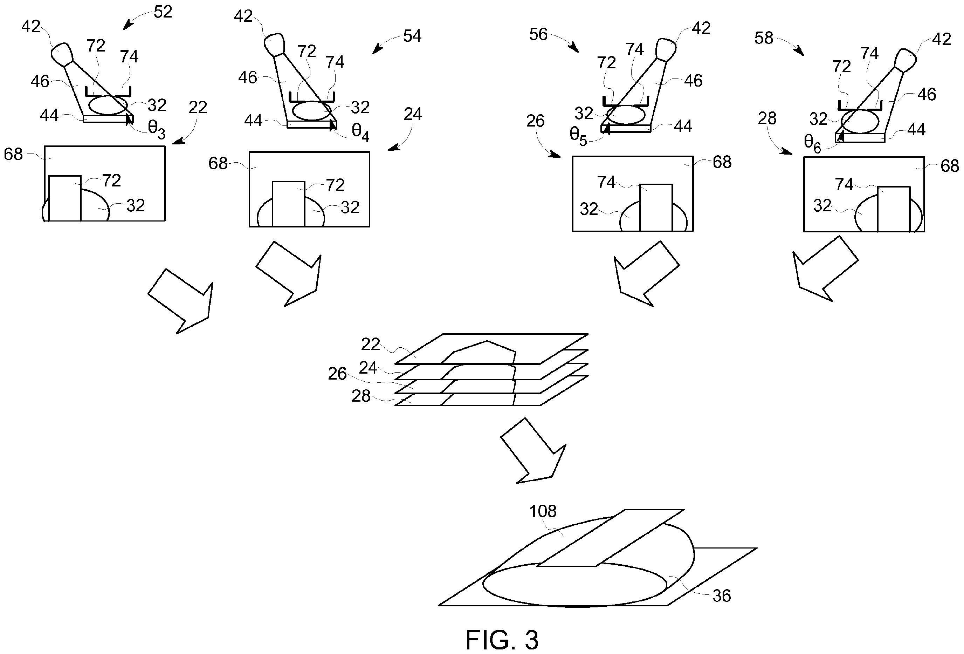

Moving to FIG. 3, in embodiments, the one or more images may be acquired in accordance with 3D tomosynthesis, which, as used herein, refers to an imaging procedure in which one or more images/projections are acquired along a partial circumference, i.e., an arc, about the object 32, as opposed to traditional computed tomography ("CT"), which usually involves the reconstruction of a 3D image from images/projections acquired along the complete circumference of an object. Thus, as will be appreciated, FIG. 3 depicts the acquisition of four (4) images 22, 24, 26, and 28 taken at four (4) positions 52, 54, 56, and 58 along an arc about the object 32 with the rays 46 intercepting the detector 44 at respective angles θ₃, θ₄, θ₅, and θ₆. While FIG. 3 depicts a 3D tomosynthesis acquisition having four (4) images, it will understood that the number of images/projections acquired during a tomosynthesis acquisition, in accordance with embodiments of the present invention, may be greater or fewer than four (4). As further shown in FIG. 3, the controller 16 may generate a three-dimensional ("3D") model 108 of the object 32 based on the acquired images 22, 24, 26, 28 with the contour 36 being based at least in part on the border/external surface of the 3D model 108. In other words, in embodiments, the contour 36 may be a 3D contour/mesh/surface estimate of the object 32.

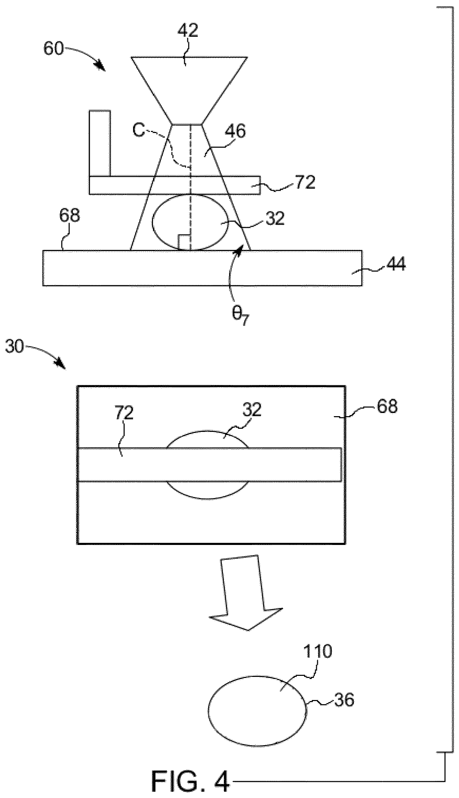

Referring now to FIG. 4, in embodiments, the one or more images may be scout images 30. As used herein the term "scout image" refers to an image that is not acquired as part of a stereo pair and/or a 3D tomosynthesis acquisition. For example, a scout image may be an initial image used to obtain a general/rough estimate of the location and/or orientation of the object 32 on the surface 68 of the detector 44. In such embodiments, the scout image 30 may be acquired with the radiation source 42 at position 60 such that the rays 46 intercept the surface 68 at an angle θ₇, e.g., positioned such that a center line C of the rays 46 is about ninety-degrees 90° to the surface 68. In such embodiments, the controller 16 may generate a two-dimensional ("2D") model 110 with the contour 36 being based at least in part on the border/external surface of the model 110. In other words, in embodiments, the contour 36 may be a 2D contour/mesh/surface estimate of the object 32.

As will be understood, in embodiments, the controller 16 may generate the contour 36 prior to any attempt to biopsy the object 32 with the biopsy apparatus 12. As such, FIG. 5 depicts the contour 36, which may be stored as a data construct in the processor 78 and/or memory device 80, as being superimposed onto the object 32 in the absence of the biopsy apparatus 12. As will be further understood, in embodiments, the controller 16 may employ a variety of methods to detect if the biopsy apparatus 12 will contact the object 32 during a biopsy attempt.

For example, as shown in FIG. 6, which also depicts the contour 36 superimposed over the object 32, the controller 16 may simulate a biopsy attempt to determine if any part/portion, e.g., a leading edge 112, of the body 92 of the biopsy apparatus 12, and/or any device attached to the apparatus 12 other than the probe 94, will touch and/or penetrate the contour 36 before the probe 94, e.g., needle, would reach the target site 96. As used herein, the term "penetrate" means to touch and/or to pass, fully or partially, through. For example, the controller 16 may use the model 102, 108, 110 to determine whether the length Lₙ of the probe 94 is as least greater than and/or equal to a length Lt of the distance between the target site 96 and a point along the contour 36 corresponding to a point on the border of the object 32 though which the probe 94 will penetrate. If Lₙ is greater than or equal to Lt, then it is likely that the probe 94 will reach the target site 96 before any point of the body 92 penetrates the contour 36. Conversely, if Lₙ is less than Lt, then it is likely that the body 92 would penetrate the contour 36 during a biopsy attempt, and in turn, contact/compress the object 32.

Accordingly, upon determining that the biopsy apparatus 12 will likely contact the object 32 during a biopsy attempt, as stated above, the controller 16 may generate a visual indicator/cue 38 (FIG. 1) and/or audio indicator/cue 40, e.g., a GUI message box, flashing light, warning tone, vocal message, etc. In embodiments, the controller 16 may prevent/restrict biopsy of the object 32 via the biopsy apparatus 12 when the controller 16 determines that a biopsy attempt will likely result in contact between the biopsy apparatus 12 and the object 32. Upon seeing and/or hearing the indicator 38, 40, and/or after being restricted by the controller 16 from attempting to biopsy the object 32, an operator of the system 10 may swap out the probe 94 for a comparable/similar probe of a different length, e.g., replace a shorter needle with a longer needle, and/or adjust the orientation of the probe 12 prior to reattempting a biopsy the object 32.

In embodiments, the controller 16 may continuously monitor the insertion of the probe 94 into the object 32 and generate the indicator 38/40 when a portion of the body 92 comes within a predetermined margin, e.g., one (1) cm of the contour 36. In other embodiments, the controller 16 may allow the object 32 to be compressed and/or contacted by the biopsy apparatus 12 by a margin, e.g., one (1) cm prior to generating the indicator 38/40.

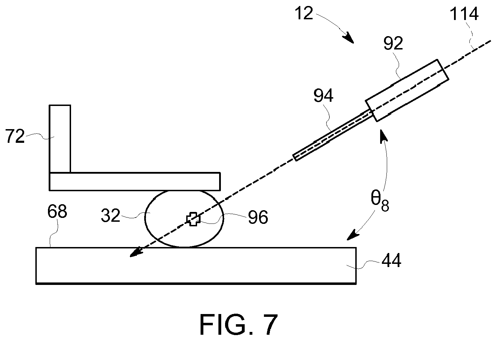

Additionally, as shown in FIG. 7, in embodiments, the controller 16 may generate a trajectory 114, i.e., a path, for the biopsy apparatus 12 to follow for biopsy of the object 32. As will be appreciated, generation of the model 102, 108, 110 may allow the controller 16 to determine a more appropriate, e.g., shorter, path for the probe 94 through the object 32 to reach the target site 96. Such a trajectory 114 may form an angle θ₈ with the surface 68 of the detector 44. In embodiments, the trajectory 114 may be a non-colliding/non-compressing/non-contacting trajectory, i.e., a trajectory that does not result in compression/contacting of the object 32 by the biopsy apparatus 12.

Finally, it is also to be understood that the system 10 may include the necessary electronics, software, memory, storage, databases, firmware, logic/state machines, microprocessors, communication links, displays or other visual or audio user interfaces, printing devices, and any other input/output interfaces to perform the functions described herein and/or to achieve the results described herein. For example, as previously mentioned, the system may include at least one processor and system memory / data storage structures, which may include random access memory (RAM) and read-only memory (ROM). The at least one processor of the system 10 may include one or more conventional microprocessors and one or more supplementary co-processors such as math co-processors or the like. The data storage structures discussed herein may include an appropriate combination of magnetic, optical and/or semiconductor memory, and may include, for example, RAM, ROM, flash drive, an optical disc such as a compact disc and/or a hard disk or drive.

Additionally, a software application that adapts the controller to perform the methods disclosed herein may be read into a main memory of the at least one processor from a computer-readable medium. The term "computer-readable medium", as used herein, refers to any medium that provides or participates in providing instructions to the at least one processor of the system 10 (or any other processor of a device described herein) for execution. Such a medium may take many forms, including but not limited to, non-volatile media and volatile media. Non-volatile media include, for example, optical, magnetic, or opto-magnetic disks, such as memory. Volatile media include dynamic random access memory (DRAM), which typically constitutes the main memory. Common forms of computer-readable media include, for example, a floppy disk, a flexible disk, hard disk, magnetic tape, any other magnetic medium, a CD-ROM, DVD, any other optical medium, a RAM, a PROM, an EPROM or EEPROM (electronically erasable programmable read-only memory), a FLASH-EEPROM, any other memory chip or cartridge, or any other medium from which a computer can read.

While in embodiments, the execution of sequences of instructions in the software application causes at least one processor to perform the methods/processes described herein, hard-wired circuitry may be used in place of, or in combination with, software instructions for implementation of the methods/processes of the present invention. Therefore, embodiments of the present invention are not limited to any specific combination of hardware and/or software.

It is further to be understood that the above description is intended to be illustrative, and not restrictive. For example, the above-described embodiments (and/or aspects thereof) may be used in combination with each other. Additionally, many modifications may be made to adapt a particular situation or material to the teachings of the invention without departing from its scope.

For example, in an embodiment, a system for management of a biopsy apparatus is provided. The system includes an imaging device and a controller. The imaging device is operative to obtain one or more images of an object to be biopsied by the biopsy apparatus. The controller is in electronic communication with the imaging device and operative to receive the one or more images; and to generate a contour of the object based at least in part on the one or more images. The controller is further operative to determine, based at least in part on the contour, if the biopsy apparatus will contact the object during biopsy of the object; and to generate an indicator upon determining that the biopsy apparatus will contact the object. In certain embodiments, the indicator is at least one of an audio indicator and a visual indicator. In certain embodiments, the controller is further operative to restrict biopsy of the object by the biopsy apparatus upon determining that the biopsy apparatus will contact the object. In certain embodiments, the contour is a two-dimensional (2D) contour. In certain embodiments, the contour is a three-dimensional (3D) contour. In certain embodiments, the controller determines that the biopsy apparatus will contact the object during biopsy of the object if a portion of a body of the biopsy apparatus will penetrate the contour before a probe guided by the biopsy apparatus would reach a target site within the object. In certain embodiments, each of the one or more images forms part of a two-dimensional (2D) pair of images of the object. In certain embodiments, the one or more images are scout images. In certain embodiments, the one or more images are acquired in accordance with a three-dimensional (3D) tomosynthesis image acquisition. In certain embodiments, the controller is further operative to generate a trajectory for the biopsy apparatus to follow for biopsy of the object. In certain embodiments, the object is a breast and the imaging system is a mammography imaging system.

Other embodiments provide for a method for management of a biopsy apparatus. The method includes obtaining one or more images of an object to be biopsied by the biopsy apparatus; and receiving the one or more images at a controller. The method further includes generating, via the controller, a contour of the object based at least in part on the one or more images; determining, via the controller, if the biopsy apparatus will contact the object during biopsy of the object based at least in part on the contour; and generating, via the controller, a contact indicator upon determining that the biopsy apparatus will contact the object. In certain embodiments, generating, via the controller, the contact indicator includes sounding an audio indicator. In certain embodiments, generating, via the controller, the contact indicator includes displaying an audio indicator. In certain embodiments, the method further includes restricting, via the controller, biopsy of the object via the biopsy apparatus upon determining that the biopsy apparatus will contact the object. In certain embodiments, each of the one or more images are scout images. In certain embodiments, obtaining one or more images of an object to be biopsied by the biopsy apparatus is accomplished via a tomosynthesis image acquisition.

Yet still other embodiments provide for a non-transitory computer readable medium storing instructions. The stored instructions adapt a controller to receive one or more images of an object to be biopsied by a biopsy apparatus; and to generate a contour of the object based at least in part on the one or more images. The stored instructions further adapt the controller to determine, based at least in part on the contour, if the biopsy apparatus will contact the object during biopsy of the object; and to generate a contact indicator upon determining that the biopsy apparatus will contact the object. In certain embodiments, each of the one or more images forms part of a two-dimensional (2D) pair of images of the object. In certain embodiments, the one or more images are scout images.

Accordingly, as will be appreciated, by determining that a biopsy apparatus will likely contact an object, i.e., compression of the object via a biopsy apparatus, during a biopsy attempt of the object, some embodiments of the present invention reduce the likelihood that a biopsy attempt will be unsuccessful, which in turn, improves the patient throughput of the encompassing imaging/biopsy system, and/or reduces patient discomfort.

Further, by generating a trajectory for a biopsy apparatus, based at least in part on a model of the object as described herein, some embodiments of the present invention reduce the risks associated with biopsy of the object by reducing the amount of distance a probe has to travel within the object in order to reach the target site.

Additionally, while the dimensions and types of materials described herein are intended to define the parameters of the invention, they are by no means limiting and are exemplary embodiments. Many other embodiments will be apparent to those of skill in the art upon reviewing the above description. The scope of the invention should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, terms such as "first," "second," "third," "upper," "lower," "bottom," "top," etc. are used merely as labels, and are not intended to impose numerical or positional requirements on their objects. Further, the limitations of the following claims are not written in means-plus-function format are not intended to be interpreted as such, unless and until such claim limitations expressly use the phrase "means for" followed by a statement of function void of further structure.

This written description uses examples to disclose several embodiments of the invention, including the best mode, and also to enable one of ordinary skill in the art to practice the embodiments of invention, including making and using any devices or systems and performing any incorporated methods. The patentable scope of the invention is defined by the claims, and may include other examples that occur to one of ordinary skill in the art. Such other examples are intended to be within the scope of the claims if they have structural elements that do not differ from the literal language of the claims, or if they include equivalent structural elements with insubstantial differences from the literal languages of the claims.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property.

Since certain changes may be made in the above-described invention, without departing from the spirit and scope of the invention herein involved, it is intended that all of the subject matter of the above description shown in the accompanying drawings shall be interpreted merely as examples illustrating the inventive concept herein and shall not be construed as limiting the invention.

## Claims

1. A system 10 for management of a biopsy apparatus 12, the system 10 comprising:
an imaging device 14 operative to obtain one or more images 18, 20, 22, 24, 26, 28, 30 of an object 32 to be biopsied by the biopsy apparatus 12;
a controller 16 in electronic communication with the imaging device 14 and operative to:
receive the one or more images 18, 20, 22, 24, 26, 28, 30;
generate a contour 36 of the object 32 based at least in part on the one or more images 18, 20, 22, 24, 26, 28, 30;
determine, based at least in part on the contour 36, if the biopsy apparatus 12 will contact the object 32 during biopsy of the object 32; and
generate an indicator 38, 40 upon determining that the biopsy apparatus 12 will contact the object 32.

2. The system 10 of claim 1, wherein the indicator 38, 40 is at least one of an audio indicator 40 and a visual indicator 38.

3. The system 10 of claim 1, wherein the controller 16 is further operative to:
restrict biopsy of the object 32 by the biopsy apparatus 12 upon determining that the biopsy apparatus 12 will contact the object 32.

4. The system 10 of claim 1, wherein the contour 36 is a two-dimensional (2D) contour.

5. The system 10 of claim 1, wherein the contour 36 is a three-dimensional (3D) contour.

6. The system 10 of claim 1, wherein the controller 16 determines that the biopsy apparatus 12 will contact the object 32 during biopsy of the object 32 if a portion 112 of a body 92 of the biopsy apparatus 12 will penetrate the contour 36 before a probe 94 guided by the biopsy apparatus 12 would reach a target site 96 within the object 32.

7. The system 10 of claim 1, wherein each of the one or more images 18, 20 forms part of a two-dimensional (2D) pair of images of the object 32.

8. The system 10 of claim 1, wherein the one or more images 30 are scout images.

9. The system 10 of claim 1, wherein the one or more images 22, 24, 26, and 28 are acquired in accordance with a three-dimensional (3D) tomosynthesis image acquisition.

10. The system 10 of claim 1, wherein the controller 16 is further operative to:
generate a trajectory 114 for the biopsy apparatus 12 to follow for biopsy of the object 32.

11. The system 10 of claim 1, wherein the object 32 is a breast and the imaging system 14 is a mammography imaging system.

12. A method for management of a biopsy apparatus 12, the method comprising:
obtaining one or more images 18, 20, 22, 24, 26, 28, 30 of an object 32 to be biopsied by the biopsy apparatus 12;
receiving the one or more images 18, 20, 22, 24, 26, 28, 30 at a controller 16;
generating, via the controller 16, a contour 36 of the object 32 based at least in part on the one or more images 18, 20, 22, 24, 26, 28, 30;
determining, via the controller 16, if the biopsy apparatus 12 will contact the object 32 during biopsy of the object 32 based at least in part on the contour 36; and
generating, via the controller 16, a contact indicator 38, 40 upon determining that the biopsy apparatus 12 will contact the object 32.

13. The method of claim 12, wherein generating, via the controller 16, the contact indicator comprises:
sounding an audio indicator 40.

14. The method of claim 12, wherein generating, via the controller 16, the contact indicator comprises:
displaying a visual indicator 38.

15. The method of claim 12 further comprising:
restricting, via the controller 16, biopsy of the object 32 via the biopsy apparatus 12 upon determining that the biopsy apparatus 12 will contact the object 32.
